Europäisches Patentamt

European Patent Office

Office européen des brevets

(19)

(11) **EP 0 941 074 B1**

(12) **EUROPEAN PATENT SPECIFICATION**

(45) Date of publication and mention
of the grant of the patent:
**21.07.2004 Bulletin 2004/30**

(21) Application number: **97946676.0**

(22) Date of filing: **05.11.1997**

(51) Int Cl.$^7$: **A61K 9/50**, A61K 31/44

(86) International application number:
**PCT/US1997/020851**

(87) International publication number:
**WO 1998/019668 (14.05.1998 Gazette 1998/19)**

(54) **DELAYED DELIVERY SYSTEM FOR ACID-SENSITIVE DRUGS**

SYSTEM ZUR VERZÖGERTEN FREISETZUNG SÄURELABILER SUBSTANZEN

MODE D'ADMINISTRATION DIFFEREE DE MEDICAMENTS SENSIBLES AUX ACIDES

(84) Designated Contracting States:
**AT BE CH DE DK ES FI FR GB GR IE IT LI LU MC
NL PT SE**

(30) Priority: **06.11.1996 US 740981**

(43) Date of publication of application:
**15.09.1999 Bulletin 1999/37**

(73) Proprietor: **Wockhardt Europe Limited
Dublin II (IE)**

(72) Inventor: **SHARMA, Vinay, K.
Long Valley, NJ 07853 (US)**

(74) Representative:
**Beresford, Keith Denis Lewis et al
BERESFORD & Co.
16 High Holborn
London WC1V 6BX (GB)**

(56) References cited:
**EP-A- 0 496 437        WO-A-96/01623**

**Description**

**Background of the Invention**

(1) Field of the Invention

The present invention relates to a drug delivery system and, more particularly, to a delayed release drug delivery system for drugs or biologically active materials (e.g., vitamins, vaccines, antibiotics, antifungal agents, muscle relaxers, mood altering drugs, and the like), and especially omeprazole. The system is capable of site-specific delivery and pulsatile (bolus) kinetics.

(2) Description of Prior Art

Omeprazole provides a powerful inhibitory action against secretion of gastric juices and can be used for treatment of gastric and duodenal ulcers. (*Lancet*, Nov. 27, 1982, pp. 1223-24). However, omeprazole is susceptible to degradation in acidic, acid reactive and neutral media, i.e., the half-life of degradation at pH 4 is less than six minutes, whereas at pH 7 it is about 14 hours. Human pharmacological studies have found that the rate of release of omeprazole can influence the total extent of absorption of the drug to the general circulation. A fully bioavailable dosage form should release the drug rapidly in the proximal part of the gastrointestinal tract.

U.S. Patent No. 4,786,505 discloses that the stability of conventional formulae of omeprazole is not satisfactory. The reference describes a dosage form including a core region containing omeprazole mixed with alkaline compounds or an alkaline salt of omeprazole mixed with an alkaline compound, or an alkaline omeprazole salt alone coated with one or more layers. The layer/layers which is/are comprised of an alkaline material separates/separate the core material from the outer layer, which is an enteric coating disposed on a subcoating. The final enteric-coated dosage form is treated to reduce water content in order to obtain stability of the drug during long-term storage.

U.S. Patent No. 4,853,230 describes a pharmaceutical preparation comprising an alkaline-reacting core containing an acid-labile pharmaceutical substance and a pH-buffering alkaline-reacting compound which provides a pH of 7-12 to the micro-environment of the acid labile compound. A subcoating layer is composed of tablet excipients, film forming compounds, and alkaline buffering compounds. Finally, an enteric coating layer surrounds the subcoating layer, wherein the subcoating layer isolates the alkaline-reacting core from the enteric coating layer such that the stability of the product is enhanced.

Omeprazole, along with many other drugs (e.g., Captopril) or biologically active materials (e.g., beta-lactams), vitamins, vaccines, antibiotics, antifungal agents, muscle relaxers, mood altering drugs, and the like can be acid sensitive. That is, their active properties can decrease significantly in the presence of acids, usually by deterioration of the drug or chemical reaction with the drug, altering its composition. The use of enteric layers, which generally comprise layers with carboxyl groups or other acid groups, can adversely affect the stability of the acid sensitive ingredients, since the acid groups on the polymers or other ingredients used in the enteric coating can directly contribute to the acid sensitizing capability of the environment of the pellets during storage.

**SUMMARY OF THE INVENTION**

The present invention provides a delayed release drug delivery system containing an acid sensitive drug which is stable at pH levels above 9.0, or biologically active material which is acid sensitive yet stable at pH levels above 9.0, such as omeprazole, for once-a-day dosage. The present invention provides a dose of an acid sensitive drug which is stable at pH levels above 9.0, or biologically active material which is acid sensitive yet stable at pH levels above 9.0, such as omeprazole comprising an alkaline core structure of high cohesiveness and integrity, capable of withstanding the pressure of attrition during a layering operation. One aspect of the present invention is to take advantage of the alkaline core structure, for optimization of release and stability of acid sensitive drugs such as with omeprazole release. Another aspect of the present invention is to provide a delayed release dosage form of an acid sensitive drug, such as omeprazole which is resistant to dissolution in acid media. Another aspect of the invention is to provide at least one sub-separation layer in the pellets adjacent to a layer containing the active acid sensitive material, the . sub-separation layer comprising a water soluble/water dispersible polymer and a pharmaceutically acceptable water soluble buffer which can provide a pH of at least 9.0 (depending upon the stabilization needs of the individual compounds), preferably at least 9.5, more preferably at least 10.0, and most preferably (in the case of omeprazole), at least 10.5 or at least 11.0 (up to 12, for example). The use of the high pH buffer materials assures that even in the presence of moisture which could cause migration of acid within the pellets, the high pH buffer would reduce any effect that migratory acid could have on the system.

A further aspect of the present invention is to provide a delayed release dosage form of an acid sensitive drug

such as omeprazole which dissolves rapidly in neutral-to-alkaline media. A still further aspect of the present invention is to provide a delayed release dosage form of omeprazole having good stability during long term storage.

The invention is defined in claim 1 and preferred embodiments thereof are defined in the dependent claims.

The present invention includes both multicompartment pellets and monocompartment pellets for the release of acid sensitive drugs or biologically active materials. The monocompartment pellets, for example, might include a substrate (e.g., nonpareil), a subseparation layer (e.g., a water-soluble or water dispersible binder or polymer (especially cellulose based polymers), such as hydroxypropyl methyl cellulose, hydroxypropyl cellulose incorporates and mixtures thereof (including with other types of water soluble or water insoluble polymers and binders, which two layers define an alkaline core structure)) and a layer of deposited omeprazole, especially as from suspensions of omeprazole or micronized omeprazole, followed by a semipermeable moisture barrier layer, and finally with an enteric barrier layer. An optional finishing layer may also be provided. The preferred alkaline structure is comprised of a core of a blend of a spheronizing/disintegrating agent such as microcrystalline cellulose and an alkaline material such as magnesium trisilicate or trisodium phosphate, although buffers or salts having the pH range of properties described above may be used.

The alkaline core structure is of high integrity and can withstand pressure of attrition during rotor layering operation and subsequent application of an aqueous suspension of omeprazole. The drug layered alkaline core structure is coated with a non-enteric moisture barrier, and then coated with a pH-dependent, enteric membrane, insoluble in simulated gastric fluid but dissolving immediately in intestinal fluid thereby releasing omeprazole by disintegration in the proximal segment of the gastrointestinal tract in a pulsatile manner.

## BRIEF DESCRIPTION OF THE DRAWINGS

A better understanding of the present invention as well as other objects and advantages thereof will become apparent upon consideration of the detailed disclosure thereof, when taken with the accompanying drawings, wherein

Figure 1 is the dissolution profile of a delayed delivery system for omeprazole at pH 5.2;
Figure 2 is the dissolution profile of a delayed delivery system for omeprazole at pH 6.0; and
Figure 3 is the dissolution profile of a delayed delivery system for omeprazole at pH 7.2.
Figure 4 is a representation of a tablet dose unit within the scope of the present invention.
Figure 5 is the drug release profile for a Multi-Compartment enteric delayed release system of the present invention with the omeprazole, using trisodium phopshate as the alkaline component.

## DETAILED DESCRIPTION OF THE INVENTION

The delayed release drug delivery system of the present invention containing omeprazole is comprised of an alkaline core structure; layered omeprazole dispersion in aqueous dispersion of water soluble binder, such as hydroxypropylmethyl cellulose, other cellulose esters, water soluble polymers and the like; a separation barrier, which may be a non-enteric moisture barrier; and a delayed release enteric barrier providing gastro-resistant behavior to deliver omeprazole in the proximal segment (pH 5-6) of the gastrointestinal tract.

Microcrystalline cellulose is the preferred binder as it assists in the absorption of water, which is used to create an alkaline micro-environment for each nonpareil central core by providing a hydroxyl ion concentration gradient. Once contact with water is made, there is migration of hydroxyl ions seeking a lower pH, moving toward omeprazole particles to enhance dissolution and to stabilize the omeprazole during dissolution in the acidic medium. A barrier of low water-solubility polymer or resin, such as ethylcellulose, cellulose acetate, or zein is provided to protect omeprazole-layered particles from subsequent delayed release membrane coats containing carboxylic functionalities during the coating operation.

I. <u>Alkaline Core Structure</u>

Basic alkaline material is selected from the group consisting of salts of strong basic cations and weak acidic anions such as $Mg^{2+}$, $Ca^{2+}$, or $Al^{3+}$ and $CO_3^{-2-3}$, $OH^-$, and metal oxides including MgO, CaO, and $Al_2O_3$, at a preferred pH of 9 or greater; organic buffers including Tris (hydroxymethyl) amino methane; and natural clays including montmorillonite and pharmaceutic necessities including sodium glycerophosphate (pH 9.5) and sodium borate (pH 9.5), ratio of omeprazole to alkaline material being from 1:1 to 1:5, preferably 1:1.5 - 1:2.5.

The alkaline core structure, in association with a spheronizing/disintegrating agent, is prepared by rotor layering nonpareils (e.g., 30/35 or 25/30 mesh, usually between 20 and 50 mesh) with a powder blend of water softenable polymer such as microcrystalline cellulose and an alkaline agent such as trisodium phosphate or magnesium trisilicate powder. A dispersion of hydroxypropyl cellulose is preferably used as a polymeric binding agent for depositing powder blend on the nonpareils. The powder blend and the binding agent are applied at appropriate powder feed rate, spray rate, air volume, and inlet temperature. Ratio of spheronizing/disintegrating agent is from 2:1 to 1:2, preferably 1:1.5 to 1.5:1.

II. Layered Drug Deposit

The layered omeprazole macroparticulates may be obtained by layering micronized omeprazole dispersion in an aqueous dispersion of water soluble/dispersible organic polymeric binders, especially cellulose-based polymers such as hydroxypropyl methylcellulose (traded as Opadry™ Y-5-7095, a registered trademark of Colorcon) or polysiloxane polymers such as Simcthicone Emulsion 30% USP on the alkaline deposit cores using rotor layering equipment.

III. Separation Barrier

The separation barrier is then created over the layered drug deposit. After the drug layering operation, the layer may be formed by any commercial process, such as for example, layering over the drug layer a dispersion of trisodium phopshate and Opadry White YS 22-7719, or a semipermeable barrier composed of water soluble or water dispersible polymers (such as cellulose-based polymers, such as cellulose esters, acetates, other derivatives and copolymers), ethylcellulose (e.g., Surelease®, Colorcon), cellulose acetate or zein.

Application of trisodium phosphate (or other pharmaceutically acceptable buffer with a pH greater than 9.0, etc. (as described below) as a separation barrier may be accomplished by any commercially available process such as suspension layering, powder layering, or compression molding the trisodium phosphate (or other buffer) with a water-soluble binder (e.g., lactose or other pharmaceutically acceptable binder, including other sugars or carbohydrates), preferably when both materials are micronized, or dissolving the buffer, e.g., the trisodium phosphate with a cosolvent (e.g., Carbowax®3350 or 8000).

The barrier thus created inhibits the interaction between dissolved omeprazole (or other acid sensitive drug or biologically active material) and carboxylate ions provided by the delayed release pH enteric barrier. Inhibition of such interaction provides protection to omeprazole from degradation and discoloration.

IV. Delayed Release Enteric Barrier

The delayed release enteric barrier is applied on the non-enteric barrier-coated material by any convenient commercial coating or layering process such as rotor-layering or fluidized bed coating, using an aqueous dispersion of the enteric coating composition, such as, for example, 30% w/w of copolymers of methacrylic acid and ethyl acrylate, plasticized with a nontoxic, pharmaceutically acceptable plasticizer, such as organic plasticizers, and especially triethyl citrate. An antiadherant, such as talc, is used to prevent agglomeration of the membrane-coated beads. As enteric coating materials, cellulose acetate phthalate (CAP), hydroxypropyl methylcellulose phthalate (HPMCP-50), polyvinyl acetate phthalate (PVAP), co-polymerized acrylic polymers such as acrylic acid or methacrylic acid/methacrylic acid methyl esters (L30D-55), or similar compounds may be used, preferably HPMCP-50 and L30D-55.

The enteric coating layer also contains pharmaceutically acceptable plasticizers, such as water-soluble triacetin, triethyl citrate, or propylene glycol. The ratio of polymer to plasticizer may be, for example, between 10:1 to 6:1, preferably being 9:1 to 7:3, and more preferably 8.5:2 to 7.5:2; enteric polymers as selected, based on minimal lag time, followed by pulsatile kinetics; and minimal reactive carboxyl groups. Therefore, the organic-based enteric polymer, HPMCP-50 and water-based enteric polymer, L30D-55, are preferred, with an apparent pKa close to 4.3 (e.g., between 4.1 and 4.5). Insofar as the reactivity of carboxyl groups is concerned, all of these enteric materials produce degradation compounds which impart a tint of purple to the omeprazole-layered alkaline core structure. Therefore, in the present invention, a semipermeable barrier is intentionally applied between the omeprazole layer and the enteric membrane, in order to prevent the degradation of omeprazole.

Preferably, the enteric membrane provides a weight gain sufficient to permit release of omeprazole after immersion in 0.1N HCl for at least two hours for enteric behavior, followed by pH 6.8 buffer, corresponding to a drug release pattern of 0% of the total omeprazole released after at least 1.5 hours of measurement in 0.1 N HCl and from 60-80% of the total omeprazole released after 45 minutes of measurement in the pH 6.8 buffer.

V. Dissolution Profiles

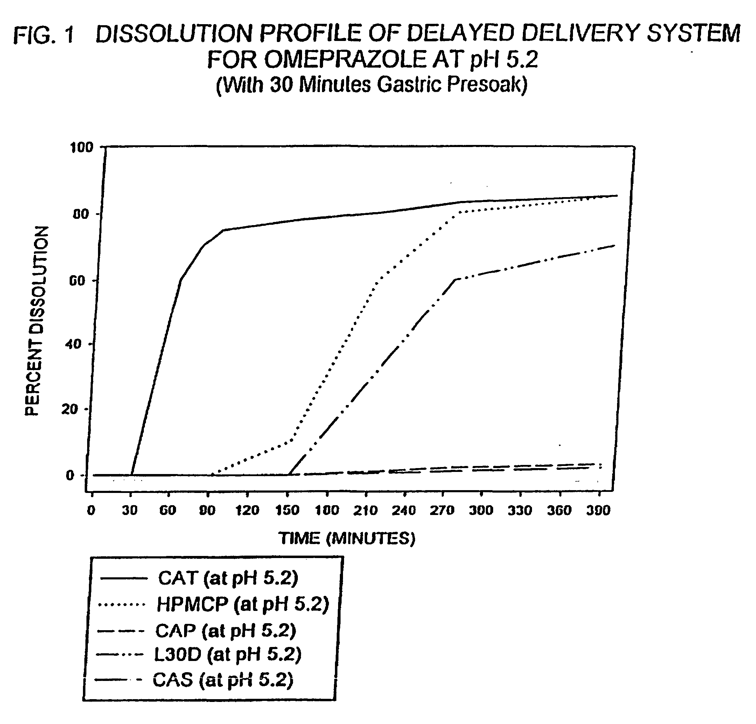

Figure 1 represents the dissolution profile of a delayed delivery system for omeprazole at pH 5.2, and distinguishes between enteric membranes with regard to lag time and kinetics of drug release. As is evident from Figure 1, only CAT (cellulose acetate trimellitate) provides an acceptable, pulsatile release kinetics. However, CAT is not acceptable in this instance. Whereas pH 5.2 is acceptable for the fasted, but not for the non-fasted stated, CAT would deliver the drug in the non-fasted stomach where the pH can approach 4-5 in some cases.

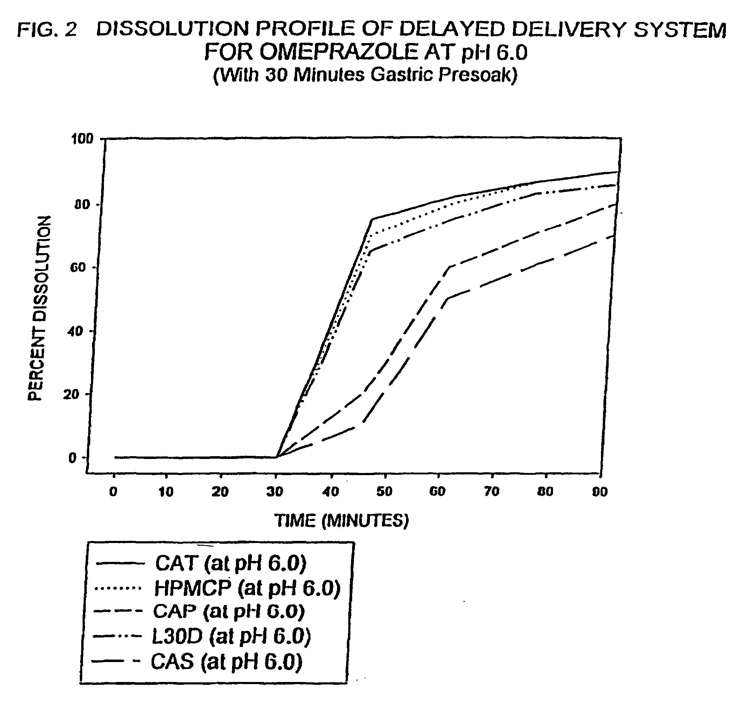

Figure 2 represents the dissolution profile of a delayed delivery system for omeprazole at pH 6.0, representative of the non-fasted state in the proximal segment of the small intestine. As indicated, HPMCP and L30D-55 provide an optimum pulsatile release profile at pH 6.0. CAT, on the other hand, while providing an optimum pulsatile release profile at pH 6.0, is not acceptable; because of its low pKa, CAT would deliver the drug in the non-fasted stomach. The other materials, CAP and CAS, do not exhibit satisfactory pulsatile release profiles and, in fact, the profiles tend to become sigmoidal in shape.

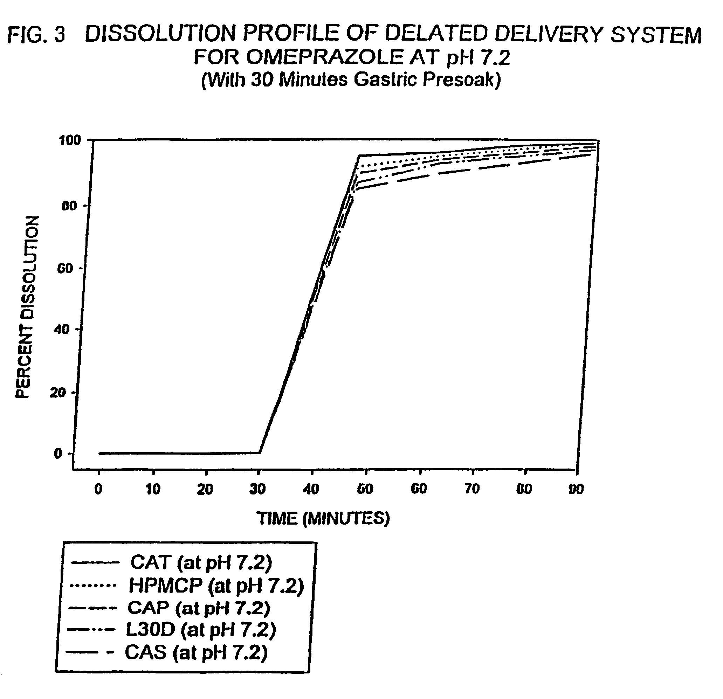

Figure 3 represents the dissolution profile of a delayed delivery system for omeprazole at pH 7.2. As shown, all enteric barrier materials provide acceptable pulsatile release kinetics; however, a pH of 7.2 approaches that of the large intestine, which is 7.5 to 8.0. Material having a higher pH, 7.2, will not deliver the drug in the proximal segment of the small intestine, where the pH ranges from 4.7 to 6.5. HPMCP and L30D-55 provide most appropriate materials for optimum pulsatile release in the targeted area.

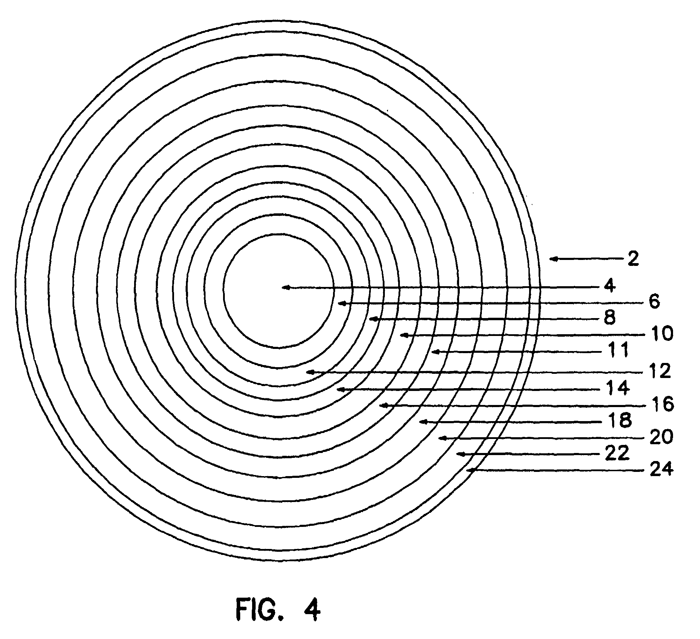

Figure 4 shows one example of a multicompartment, enteric delayed release system dose unit of the present invention **2** comprising a substrate **4** having alternating subseparation layers **6, 8, 10** and **11,** and drug layers **12, 14, 16** and **18.** A separation layer **20** overlays all of the previous layers and is in turn covered with an enteric membrane **22** and a finish coat **24.**

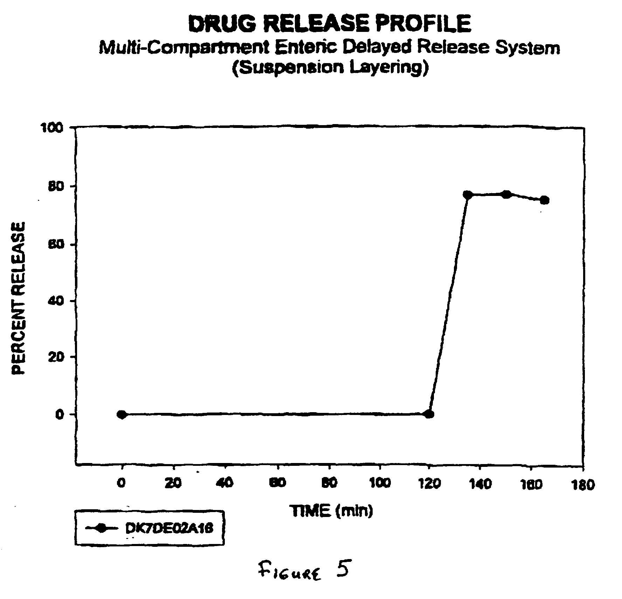

Figure 5 shows a drug release profile of a multicompartment, enteric delayed release system like that of Figure 4. As can be seen from the graph, the initial release of the omeprazole was delayed for approximately two hours, with a rapid and steady release occurring quickly over an approximately ten minute period.

**EXAMPLES**

The specific nature of the composition of the present invention will be more fully apparent from consideration of the following specific examples of preferred embodiments thereof. In the examples, as in the preceding description, all parts and percentages are given by weight unless otherwise indicated.

Example 1

| Preparation of Alkaline Core Structure | |
|---|---|
| Nonpareils 30/35 mesh | 750 g. |
| Magnesium Trisilicate | 1500 g. |
| Microcrystalline Cellulose | 1500 g. |
| Klucel (6% w/w/ = 4175) | 250.5g. |
| Total: | 4000.5g. |

The preparation of the alkaline core is achieved by using a blend of a spheronizing agent such as microcrystalline cellulose and an alkaline material such as magnesium trisilicate (preferably, in a 50/50 ratio) which is powder-layered on 30/35 mesh non-pareils, using a 12" insert in a fluid bed granulator dryer (traded as FLM-15 EX by Vector, Corporation, Marion, Ia.). Particle size analysis is as follows:

| Mesh | 14 | 16 | 18 | 20 | 25 | 30 | Pan |
|---|---|---|---|---|---|---|---|
| Percent Retained | 2 | 22 | 64 | 10 | 1 | 1 | 4 |

The process yield was 90%. The theoretical potency of magnesium trisilicate is 375%.

Example 2

| Composition of Suspension for Layered Drug Deposit | |
|---|---|
| Omeprazole | 111 g. |
| Opadry Y-5-7095 | 44.4g. |
| Water, deionized | 954.6g. |

(continued)

| Composition of Suspension for Layered Drug Deposit | |
|---|---|
| Total: | 1110g. |

Example 3

| Composition of Layered Drug Deposit | | |
|---|---|---|
| | Solids, g. | %, w/w |
| Omeprazole | 111 | 9.607 |
| Opadry Y-5-7095 | 44.4 | 3.843 |
| Alkaline Core Structure | 1000.0 | 86.550 |
| | 1155.4 | 100.000 |

The omeprazole layer dispersion is prepared by weighing purified water into a tared container equipped with a Lightnin Mixer with impeller. With vigorous mixing, hydroxypropyl methylcellulose (Opadry Y-5-7095) is dispersed in water to prepare a smooth paste. Then, remaining water is added to prepare a clear dispersion. To this dispersion, omeprazole (111 g.) is added, to obtain a final omeprazole concentration of 10% w/w. The omeprazole suspension is suspension-layered on magnesium trisilicate pellets, using a 12" rotor insert in a fluid bed granulator dryer (traded as FLM-15 EX by Vector Corporation, Inc.).

Example 4

Preparation of Non-Enteric Moisture Barrier

To the drug deposit core, a 3% weight gain is applied, using a water-based dispersion of ethylcellulose (traded as Surelease[R] by Colorcon, Inc.; Pa.). this is accomplished by using a 12" rotor insert in the FLM-15 EX fluid bed granulator dryer.

Example 5

| Delayed Release Enteric Barrier(s) | |
|---|---|
| Eudragit L30D-55 | 594.3 g. |
| Talc, U.S.P. | 35.66 g. |
| Triethyl Citrate | 35.66 g. |
| Water, Deionized | 334.33 g. |
| Total: | 1,000.00 g. |

A water-based dispersion of Eudragit L30D-55, plasticized with triethyl citrate is applied to provide a weight gain of 15%. An antiadherant talc is used to prevent agglomeration of the membrane-coated beads.

| Cellulose Acetate Phthalate | 100 g. |
|---|---|
| Diethyl Phthalate | 25.08 g. |
| Water, Deionized | 26.25 g. |
| Acetone, N.F. | 848.75 g. |
| Total: | 1000.00 g. |

An organic-based dispersion of cellulose acetate phthalate, plasticized with diethyl phthalate, is applied to provide a weight gain of 15%

| Cellulose acetate trimellitate, N.F. | 100.08 g. |
|---|---|
| Triacetin | 25.08 g. |
| Water, Deionized | 26.25 g. |
| Acetone, N.F. | 848.75 g. |
| Total: | 1000.00 g |

An organic-based dispersion of cellulose acetate trimellitate, plasticized with triacetin, is applied to provide a weight gain of 15%.

| Hydroxypropyl methylcellulose phthalate (HPMCP-50) | 100.0 g. |
|---|---|
| Acetylated monoglyceride (Myvacet 9-45) | 15.0 g. |
| Water, Deionized | 44.25 g. |
| Acetone, N.F. | 840.75 g. |
| | 1000.00 g. |

An organic-based dispersion of HPMCP-50, plasticized with Myvacet 9-45, is applied to provide a weight gain of 15%. A supercoat of 2% weight gain, using Opadry 7065 (10% w/w dispersion) is used to finish off the coating operation.

The potency of omeprazole in imeprazole beads will be as follows:

Example 6

| Potencies at Various Stages Coating. Percent | |
|---|---|
| Drug layered drug deposit | = 9.607 |
| Sub-coated drug layered beads (2% weight gain based on alkaline core structure) | = 9.444 |
| Semipermeable barrier coat (1% weight gain) | = 9.364 |
| Enteric-coated drug layered beads (15% weight gain) | = 8.312 |
| Super-coated Enteric-coated drug-layered beads (2% weight gain) | = 8.189 |

For 20 mg. dosage, therefore, 244 mg. of pellets are required.

Example 7

| Composition of alkaline core structure | |
|---|---|
| Nonpareils 25/30 mesh | 750.0 g |
| Trisodium Phosphate (1.5% w/w) | 31.68 g |

(continued)

| Composition of alkaline core structure | |
| --- | --- |
| Opadry White YS-22-7719 (16.39% w/w) | 21.32 g |
| TOTAL: | 803.00 g |

The preparation of the separation layer is achieved by suspension layering on 25/30 mesh nonpareil seeds a dispersion of trisodium phospahe and Opadry White YS-22-7719 in purified water, using a twelve inch insert in a fluidized bed granulator dryer (FLM-15-ES by Vector Corp., Marion, Ia). The total quantity to be applied for five separation layers is 1289.4 grams.

Example 8

| Composition of drug layer | |
| --- | --- |
| Omeprazole USP, micronized | 165.0 g |
| Lactose monohydrate fine powder | 562.9 g |
| Talc, USP | 29.6 g |

The powdered drug layer consists of 165.0 grams of micronized Omeprazole USP and 592.5 grams Lactose monohydrate fine powder (micronized) and 29.6 grams of talc. The particle size of omeprazole and the excipients is below 20 microns. The theoretical quantity to be applied to the multilayered separation layered pellets is 757.5 grams.

Example 9

Theoretical quantity to be applicd to the individual separation and drug layers as a total weight of a batch preparation.

| Sublayer 1 To subsrate | 214.9 g |
| --- | --- |
| Drug layer 1 To separation layered pellets | 189.4 g |
| Sublayer 2 To drug layered pellets | 214.9 g |
| Drug layer 2 to separation layered pellets | 189.4 g |
| Sublayer 3 to drug layered pellets | 214.9 g |
| Drug layer 3 To separation layered pellet | 189.4 |
| Sublayer 4 to drug layered pellets | 214.9 g |
| Drug layer 4 To separation layered pellet | 189.4 |
| Sublayer 5 to drug layered pellets | 429.8 g |

The total theoretical to be applied to the five separation layers and the four drug layers comprises 1289.4 grams and 757.6 grams, respectively.

Example 10

| Composition of alkaline core structure | |
| --- | --- |
| Nonpareils 25/30 mesh | 750.0 g |
| Trisodium Phosphate (1.06% w/w) | 5.28 g |
| Opadry White YS-22-7719 (16.38% w/w) | 35.22 g |
| TOTAL: | 790.5 g |

The preparation of the separation layer is achieved by suspension layering on 25/30 mesh nonpareil seeds a dispersion of trisodium phosphate and Opadry White YS-22-7719 in purified water, using a twelve inch insert in a fluidized bed granulator dryer (FLM-15-ES by Vector Corp., Marion, Ia). The total quantity to be applied for the alkaline core structure is 214.9 grams.

Example 11

| Composition of drug layer | |
|---|---|
| Omeprazole USP, | 135.35 g |
| Opadry Y-5-7095 | 133.35 g |
| Simethicone Emulsion 30%, USP | 0.606 g |
| Total | 267.306 g |

The suspension of the layered drug deposit is composed of micronized drug particles in a dispersion of Simethicone emulsion 30% USP and Opadry White Y-5-7095 in purified water. Total theoretical quantity to be applied to the four drug layers is 1333.5 grams. The Omeprazole suspension is suspension layered on the alkaline core structure (composed of water-soluble trisodium phosphate), using a twelve inch rotor insert in a fluid bed granulation dryer (FLM-15 ES, Vector Corporation, Marion, Ia).

**Claims**

1. A drug delivery system for omeprazole comprising a plurality of pellets wherein each pellet comprises:

    a) a core comprising an alkaline material,

    b) multiple layers of omeprazole surrounding the core;

    c) sub-separation layers adjacent to the omeprazole layers; and

    d) at least one enteric layer comprising an enteric film forming polymer and a pharmaceutically acceptable plasticizer.

2. The drug delivery system of claim 1 wherein the core comprises a basic alkaline material and a spheronizing agent.

3. The drug delivery system of claim 1 wherein the sub-separating layer comprises a pharmaceutically acceptable buffer which provides a pH of at least 9.0.

4. The drug delivery system of any of claims 1 to 3 wherein the alkaline material is elected from the group consisting of salts of strong basic cations and weak acidic anions; organic buffers; natural clays; and sodium borate.

5. The drug delivery system of any of claims 1 to 4 wherein the alkaline material is trisodium phosphate or magnesium trisilicate.

6. The drug delivery system of claim 4 wherein the organic buffer is tris (hydroxymethyl) amino methane.

7. The drug delivery system of any of claims 1-6 wherein the ratio of omeprazole to alkaline material is 1:1 to 1:5.

8. The drug delivery system of any of claims 1-7 wherein the pellets comprise at least three distinct omeprazole layers separated by sub-separation layers.

9. The drug delivery system of any of claims 1-8 wherein the sub-separation layers comprise water-soluble or water dispersible cellulosic polymers.

10. The drug delivery system of any of claims 1-9 wherein each of the pellets further comprises a non-enteric moisture barrier comprising water-insoluble semi-permeable polymeric membranes.

11. The drug delivery system of any of claims 1-10 wherein the sub-separation layer is a water-insoluble cellulosic polymer, cellulose acetate or zein.

12. The drug delivery system of any of claims 1-11 wherein the enteric layer comprises enteric film former and a water-soluble plasticizer.

13. The drug delivery system of any of claims 1-12 wherein the enteric coating is selected from the group consisting of acrylic and resin lacquers, anionic polymers of methacrylic acid, methacrylic acid esters or cellulose phthalic acid ester derivatives.

14. The drug delivery system of claim 11 or claim 12 when dependent on claim 11, wherein the plasticizer is triacetin, triethyl citrate or propylene glycol.

15. The drug delivery system of any of claims 1-14, wherein the system is insoluble in a gastric environment.

16. A drug delivery system of any of claims 1-15 wherein the enteric membrane provides a weight gain sufficient to permit release of omeprazole after immersion in 0.1N HCl for at least two hours for enteric behavior, followed by pH 6.8 buffer, corresponding to a drug release pattern of 0% of the total omeprazole released after at least 1.5 hours of measurement in 0.1 N HCl and from 60-80% of the total omeprazole released after 45 minutes of measurement in the pH 6.8 buffer.

17. The drug delivery system of claim 8 wherein the pellets comprise four distinct omeprazole layers and a sub-separation layer between the core and the first omeprazole layer.

**Patentansprüche**

1. Arzneimittel-Zuführsystem für Omeprazol, mit mehreren Kügelchen, wobei jedes Kügelchen aufweist:

   a) einen ein Alkalimaterial umfassenden Kern,
   b) mehrere Schichten Omeprazol, die den Kern umgeben;
   c) Sub-Trennschichten, die an die Omeprazolschichten angrenzen; und
   d) mindestens eine enterische Schicht, die ein einen enterischen Film bildendes Polymer und einen pharmazeutisch akzeptablen Weichmacher aufweist.

2. Arzneimittel-Zuführsystem nach Anspruch 1, wobei der Kern ein basisches Alkalimaterial und ein Spheronizing-Mittel aufweist.

3. Arzneimittel-Zuführsystem nach Anspruch 1, wobei die Sub-Trennschicht einen pharmazeutisch akzeptablen Puffer mit einem pH-Wert von wenigstens 9,0 aufweist.

4. Arzneimittel-Zuführsystem nach einem der Ansprüche 1 bis 3, wobei das Alkalimaterial aus einer Gruppe ausgewählt ist, die aus Salzen mit stark basischen Kationen und schwach sauren Anionen; organischen Puffern; natürlichen Tonen; und Natriumborat besteht.

5. Arzneimittel-Zuführsystem nach einem der Ansprüche 1 bis 4, wobei das Alkalimaterial Trinatriumphosphat oder Magnesiumtrisilicat ist.

6. Arzneimittel-Zuführsystem nach Anspruch 4, wobei der organische Puffer Tris(hydroxymethyl)aminomethan ist.

7. Arzneimittel-Zuführsystem nach einem der Ansprüche 1 bis 6, wobei das Verhältnis von Omeprazol zu dem Alkalimaterial zwischen 1:1 und 1:5 liegt.

8. Arzneimittel-Zuführsystem nach einem der Ansprüche 1 bis 7, wobei die Kügelchen wenigstens drei verschiedene Omeprazolschichten aufweisen, die durch die Sub-Trennschichten getrennt sind.

9. Arzneimittel-Zuführsystem nach einem der Ansprüche 1 bis 8, wobei die Sub-Trennschichten wasserlösliche oder wasserdispergierbare Cellulosepolymere aufweisen.

10. Arzneimittel-Zuführsystem nach einem der Ansprüche 1 bis 9, wobei jedes Kügelchen ferner eine nicht-enterische Feuchtigkeitsbarriere mit wasserunlöslichen, semipermeablen Polymermembranen aufweist.

11. Arzneimittel-Zuführsystem nach einem der Ansprüche 1 bis 10, wobei die Sub-Trennschicht ein wasserunlösliches Cellulosepolymer, Celluloseacetat oder Zein ist.

12. Arzneimittel-Zuführsystem nach einem der Ansprüche 1 bis 11, wobei die enterische Schicht ein Mittel zum Bilden eines enterischen Films und einen wasserlöslichen Weichmacher aufweist.

13. Arzneimittel-Zuführsystem nach einem der Ansprüche 1 bis 12, wobei die enterische Beschichtung aus einer Gruppe ausgewählt ist, die aus Acryl- und Harzlacken, anionischen Polymeren einer Methacrylsäure, Methacrylsäureestern oder Cellulose-Phthalsäureester-Derivaten besteht.

14. Arzneimittel-Zuführsystem nach Anspruch 11 oder Anspruch 12, wenn abhängig von Anspruch 11, wobei der Weichmacher Triacetin, Triethylcitrat oder Propylenglycol ist.

15. Arzneimittel-Zuführsystem nach einem der Ansprüche 1 bis 14, wobei das System in einer Magenumgebung unlöslich ist.

16. Arzneimittel-Zuführsystem nach einem der Ansprüche 1 bis 15, wobei die enterische Membran eine Gewichtssteigerung bewirkt, die ausreicht, um nach Eintauchen in 0,1N HCl für wenigstens zwei Stunden für enterisches Verhalten, gefolgt von einem pH 6,8-Puffer, ein Freisetzen von Omeprazol zu ermöglichen, das einem Arzneimittel-Freisetzungsmuster entspricht, gemäß dem 0% des gesamten Omeprazols nach wenigstens 1,5 Stunden Messung in 0,1N HCl und zwischen 60 und 80% des gesamten Omeprazols nach 45 Minuten Messung in dem pH 6,8-Puffer freigesetzt ist.

17. Arzneimittel-Zuführsystem nach Anspruch 8, wobei die Kügelchen vier verschiedene Omeprazolschichten und eine Sub-Trennschicht zwischen dem Kern und der ersten Omeprazolschicht aufweisen.

**Revendications**

1. Un système de libération de médicament pour l'oméprazole, comprenant un ensemble de pastilles dans lequel chaque pastille comprend :

   a) un noyau comprenant une substance alcaline ;
   b) plusieurs couches d'oméprazole entourant le noyau ;
   c) des sous-couches de séparation adjacentes aux couches d'oméprazole ; et
   d) au moins une couche entérique comprenant un polymère formant un film entérique et un plastifiant pharmaceutiquement acceptable.

2. Le système de libération de médicament selon la revendication 1, dans lequel le noyau comprend une substance alcaline basique et un agent de sphéronisation.

3. Le système de libération de médicament selon la revendication 1, dans lequel la sous-couche de séparation comprend un tampon pharmaceutiquement acceptable qui permet d'avoir un pH d'au moins 9,0.

4. Le système de libération de médicament selon l'une quelconque des revendications 1 à 3, dans lequel la substance alcaline est choisie parmi le groupe constitué de sels de cations basiques forts et d'anions acides faibles ; des tampons organiques ; des argiles naturelles ; et du borate de sodium.

5. Le système de libération de médicament selon l'une quelconque des revendications 1 à 4, dans lequel la substance alcaline est le phosphate de trisodique ou le trisilicate de magnésium.

6. Le système de libération de médicament selon la revendication 4, dans lequel le tampon organique est le tris (hydroxyméthyl)aminométhane.

7. Le système de libération de médicament selon l'une quelconque des revendications 1 à 6, dans lequel le rapport de l'oméprazole à la substance alcaline est compris dans la plage allant de 1:1 à 1:5.

8. Le système de libération de médicament selon l'une quelconque des revendications 1 à 7, dans lequel les pastilles comprennent au moins trois couches distinctes d'oméprazole séparées par des sous-couches de séparation.

9. Le système de libération de médicament selon l'une quelconque des revendications 1 à 8, dans lequel les sous-couches de séparation comprennent des polymères cellulosiques solubles ou dispersibles dans l'eau.

10. Le système de libération de médicament selon l'une quelconque des revendications 1 à 9, dans lequel chacune des pastilles comprend en outre une barrière contre l'humidité non entérique comprenant des membranes polymères semi-perméables insolubles dans l'eau.

11. Le système de libération de médicament selon l'une quelconque des revendications 1 à 10, dans lequel la sous-couche de séparation est un polymère cellulosique insoluble dans l'eau, un acétate de cellulose ou la zéine.

12. Le système de libération de médicament selon l'une quelconque des revendications 1 à 11, dans lequel la couche entérique comprend un agent de formation de film entérique et un plastifiant soluble dans l'eau.

13. Le système de libération de médicament selon l'une quelconque des revendications 1 à 12, dans lequel l'enrobage entérique est choisi dans le groupe constitué des laques acryliques et de résine, des polymères anioniques de l'acide méthacrylique, des esters de l'acide méthacrylique ou des dérivés d'ester de l'acide phtalique cellulosique.

14. Le système de libération de médicament selon la revendication 11 ou 12 lorsqu'il dépend de la revendication 11, dans lequel le plastifiant est la triacétine, le citrate de triéthyle ou le propylène glycol.

15. Le système de libération de médicament selon l'une quelconque des revendications 1 à 14, dans lequel le système est insoluble dans un milieu gastrique.

16. Le système de libération de médicament selon l'une quelconque des revendications 1 à 15, dans lequel la membrane entérique apporte une augmentation de poids suffisante pour permettre la libération de l'oméprazole après une immersion dans 0,1 N de HCl pour un comportement entérique pendant au moins deux heures, puis dans un tampon de pH 6,8, correspondant à un profil de libération du médicament de 0 % de l'oméprazole total libéré après au moins 1,5 heure de mesure dans 0,1 N de HCl, et de 60 à 80 % de l'oméprazole total libéré après 45 minutes de mesure dans un tampon de pH 6,8.

17. Le système de libération de médicament selon la revendication 8, dans lequel les pastilles comprennent quatre couches distinctes d'oméprazole, et une sous-couche de séparation entre le noyau et la première couche d'oméprazole.

## FIG. 1 DISSOLUTION PROFILE OF DELAYED DELIVERY SYSTEM FOR OMEPRAZOLE AT pH 5.2
### (With 30 Minutes Gastric Presoak)

FIG. 2  DISSOLUTION PROFILE OF DELAYED DELIVERY SYSTEM
FOR OMEPRAZOLE AT pH 6.0
(With 30 Minutes Gastric Presoak)

# FIG. 3 DISSOLUTION PROFILE OF DELATED DELIVERY SYSTEM FOR OMEPRAZOLE AT pH 7.2
## (With 30 Minutes Gastric Presoak)

FIG. 4

# DRUG RELEASE PROFILE
## Multi-Compartment Enteric Delayed Release System
### (Suspension Layering)

FIGURE 5